(19) Europäisches Patentamt
European Patent Office
Office européen des brevets

(11) Veröffentlichungsnummer: **0 126 923**
**B1**

(12) # EUROPÄISCHE PATENTSCHRIFT

(45) Veröffentlichungstag der Patentschrift: **12.08.87**

(51) Int. Cl.⁴: **C 07 C 67/343**, C 07 C 69/716, C 07 D 211/32

(21) Anmeldenummer: **84104017.3**

(22) Anmeldetag: **10.04.84**

(54) **Verfahren zur Herstellung von 4-substituierten Acetessigsäurederivaten.**

(30) Priorität: **26.05.83 CH 2878/83**

(43) Veröffentlichungstag der Anmeldung: **05.12.84 Patentblatt 84/49**

(45) Bekanntmachung des Hinweises auf die Patenterteilung: **12.08.87 Patentblatt 87/33**

(84) Benannte Vertragsstaaten: **AT BE CH DE FR GB IT LI NL SE**

(56) Entgegenhaltungen: **EP-A-0 018 894**

**Die Akte enthält technische Angaben, die nach dem Eingang der Anmeldung eingereicht wurden und die nicht in dieser Patentschrift enthalten sind.**

(73) Patentinhaber: **LONZA AG, Gampel/Wallis (CH)**

(72) Erfinder: **Previdoli, Felix, Dr., Haus Alpenflug Ried bei Brig, (Kanton Wallis) (CH)**
Erfinder: **Tenud, Leander, Dr., Balfrinstrasse 23, Visp (Kanton Wallis) (CH)**

(74) Vertreter: **von Füner, Alexander, Dr., Patentanwälte v. Füner, Ebbinghaus, Finck Mariahilfplatz 2 & 3, D-8000 München 90 (DE)**

**EP 0 126 923 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft ein Verfahren zur Herstellung von 4-substituierten Acetessigsäurederivaten aus den entsprechenden Acetessigsäurederivaten.

Es ist bekannt, dass bei der Alkylierung von Acetessigestern die Alkylierung am $C_2$ stattfindet. Soll die Alkylierung aber am $C_4$ stattfinden, muss entweder das sogenannte Dianion (Dicarbanion) des Acetessigesters gebildet werden [J.Org.Chem. 29 (1964) S.3249; J.Am.Chem.Soc. 92 (1970) S.6702; J.Am.Chem. Soc. 96 1(1974) S.1032] oder man geht vom Lithiumsalz des entsprechenden 3-Pyrrolidonderivates des Acetessigsäureesters aus [JP-Pat.publ. 31608(1974)].

Bildet man das Dianion mit Hilfe von Kaliumamid in flüssigem Ammoniak, werden schlechte Ausbeuten an Dianion erhalten und somit auch schlechte Ausbeuten [kleiner als 37 %] an gewünschtem 4-substituierten Produkt [J.Org.Chem.29 (1964) S.3249]. Erst wenn man das Dianion in zwei Stufen erzeugt, nämlich zuerst mittels Natrium- oder Kaliumhydrid das Monoanion bildet und dieses mit n-Butyllithium in das Dianion überführt, werden bessere Ausbeuten erzielt. Diese liegen aber auch in diesem Fall nicht höher als etwas über 80 %. Neben den gewünschten 4-substituierten Produkten fallen auch 2-substituierte Produkte an, die sich nur schwer abtrennen lassen. Alkyliert man Acetessigsäurederivate nach dem Verfahren der JP-Pat.publ. 31608, muss zunächst das Lithiumsalz mit Hilfe des teuren Butyllithium erzeugt werden. Dabei resultieren Ausbeuten, die in der Größenordnung derer liegen, die beim Arbeiten mit Dianion erzielt werden.

Vergleichbar mit dem oben angeführten Verfahren nach. J. org. Chem. 29 (1964), 3249 ff. ist auch das Verfahren gemäß EP-Anmeldung 18 894, wobei Acetessigsäureester mit in situ hergestelltem Natriumamid mit flüssigem Ammoniak behandelt wird und danach durch Behandlung mit 1-Chloro-3-methyl-2-buten in dem entsprechenden 7-Methyl-3-oxo-6-octensäuremethylester übergeführt wird.

Mechanistisch betrachtet wird dort mit Natriumamid und dem Acetessigester ein Dianion der Formel

$$C^{\ominus}H_2 - \overset{O}{\overset{\|}{C}} - C^{\ominus}H_2 - \overset{O}{\overset{\|}{C}} - OR$$

erzeugt. Die vollständigkeit dieser Deprotonierung ist nun verantwortlich für das Resultat der Weiterreaktion mit der entsprechenden Halogenverbindung.

Die schlechten Resultate aus J. org. Chem. 29 (1964), 3250, Tabelle 1, mit Ausbeuten von maximal 37 % lassen vermuten, daß eben diese Dianionbildung nicht vollständig erfolgte.

Nicht überraschend ist daher auch die in der gleichen Größenordnung liegende Ausbeute von 38,4 % aus der EP-Anmeldung 18 894.

Arbeitet man über das Dianion nach dem beschriebenen nachteiligen Verfahren, ist es offenbar nicht möglich, Ausbeuten über 40 % zu erreichen.

Aufgabe der vorliegenden Erfindung war es, ein technisch einfach durchführbares Verfahren zu finden, das die Nachteile der bekannten Verfahren nicht aufweist.

Diese Aufgabe wurde mit einem Verfahren nach Patentanspruch 1 gelöst.

Das erfindungsgemäße Verfahren besteht aus mehreren Stufen. Die Produkte der einzelnen Stufen können isoliert werden. Es ist aber duch möglich, die Stufen ohne Isolation der jeweiligen Produkte als Eintopfverfahren zu fahren.

In der ersten Stufe wird das Enaminocarbonssäurederivat nach bekannten Methoden der Enaminherstellung gebildet

Als sekundäres Amin kommen beispielsweise Dimethylamin, Diäthylamin, Piperidin, Morpholin, Pyrrolidin, Azetidin in Frage. Vorzugsweise wird Pyrrolidin verwendet.

Anschliessend wird das Enaminocarbonsäurederivat mit Natriumamid in flüssigem Ammoniak in das Natriumsalz übergeführt. Dabei kann das Lösungsmittel entfernt oder das Enaminocarbonsäurederivat als Lösung eingesetzt werden.

Die Natriumamidmenge wird so gewählt, dass vorzugsweise pro Mol Ausgangsprodukt 1,0 bis 1,4 Mol eingesetzt werden.

Die Menge flüssigen Ammoniaks ist nicht kritisch, vorteilhaft werden jedoch pro Mol Ausgangsprodukt 20 bis 30 Mol $NH_3$ verwendet.

Nach der Bildung des Natriumsalzes bestehen drei Varianten des weiteren Vorgehens. Eine Variante besteht darin, die weitere Umsetzung direkt in flüssigem Ammoniak durchzuführen. Eine andere Variante besteht darin, das Ammoniak abzudestillieren und die weitere Umsetzung in einem organischen Lösungsmittel durchzuführen. Es ist aber auch möglich, das Ammoniak im Reaktionsgefäss zu belassen und zusätzlich ein organisches Lösungsmittel zu verwenden.

Die eingesetzten Lösungsmittel müssen einen Schmelzpunkt unter der Reaktionstemperatur haben und gegenüber starken Basen unreaktiv sein. Ferner muss die natriumorganische Verbindung darin löslich sein.

Als dafür zweckmässige Lösungsmittel kommen aromatische Kohlenwasserstoffe wie Benzol, Toluol, Xylol usw., aliphatische Kohlenwasserstoffe wie Hexan, Pentan, Cyclohexan usw., Äther wie Diäthyläther, Dioxan, Tetrahydrofuran usw., ferner Dimethylsulfoxid, Dimethylformamid, Hexamethylphosphorsäuretriamid, Pyridin, substituierte Pyridine usw. in Frage.

Als Halogenverbindung der Formel

$R_1CH_2X$ oder $R_1R_2CHX$,

worin $R_1$ und $R_2$ Alkyl-, Alkinyl-, Arylreste und X Chlor, Brom oder Jod bedeuten, können vorzugsweise genannt werden:

Methylchlorid, Methylbromid, Methyljodid, Äthylchlorid, Äthylbromid, Propylbromid, Allylchlorid, Isopropylbromid, Isopropyljodid, Butyljodid, Butylbromid, Propargylbromid, Benzylchlorid, Benzylbromid.

Nach der Alkylierung wird durch Hydrolyse, vorzugsweise mit Hilfe einer Mineralsäure, wie z. B. Salzsäure oder Schwefelsäure, das Endprodukt in Freiheit gesetzt. Dabei wird zweckmässig in Gegenwart eines mit Wasser nicht mischbaren Lösungsmittels, vorzugsweise im, bei der Alkylierung verwendeten Lösungsmittel, gearbeitet.

Für den Fall, dass neben 4-Substitutionsprodukten auch kleine Mengen 2-Substitutionsprodukte auftreten, können letztere mit Wasser selektiv zu 2-substituierten Acetessigsäurederivaten hydrolisiert und leicht abgetrennt werden. Anschliessend werden die 4-Substitutionsprodukte mit wässriger Säure zu den entsprechenden 4-substituierten Acetessigsäurederivaten hydrolisiert.

Die Bildung des Natriumsalzes und die Alkylierung kann bei Temperaturen von -60 bis +40°C durchgeführt werden. Wird bei höheren Temperaturen und/oder mit gasförmigem Alkylierungsmittel gearbeitet, wird Druck angewendet.

Gasförmige Alkylierungsmittel können auch in einem Lösungsmittel gelöst angewendet werden.

**Beispiel 1**

3-Oxopentansäuremethylester

116 g Acetessigsäuremethylester und 75 g Pyrrolidin wurden in 350 ml Toluol am Wasserabscheider gekocht, bis die theoretische Menge Wasser abgeschieden war. Diese Lösung wurde bei -40°C zu einer Suspenson von 47 g Natriumamid in 800 ml flüssigem Ammoniak zugetropft. Man entfernte das Ammoniak, indem man die Temperatur der Reaktionslösung auf -20°C ansteigen liess und tropfte dann bei dieser Temperatur eine Lösung von 114 g Methylbromid in 500 ml Toluol zu. Die Reaktionslösung wurde filtriert und das Toluol am Rotationsverdampfer entfernt. Nach Zugabe von 700 ml Methylenchlorid und 102 g konzentrierter Salzsäure zum Rückstand wurde während 1 Stunde bei Raumtemperatur hydrolisiert. Die beiden Phasen wurden getrennt, die organische Phase wurde getrocknet und das Lösungsmittel entfernt.

Man erhielt 120 g 3-Oxopentansäuremethylester (92 %).

**Beispiel 2**

5-Phenyl-3-oxopentansäure-tert-butylester

Man verfuhr analog wie in Beispiel 1 beschrieben. Bei -40 bis -50°C wurden dann 133 g Benzylchlorid in 500 ml Toluol zugetropft. Es wurde dann durch Zugabe von 200 ml Wasser und 102 g konzentrierter Salzsäure bei Raumtemperatur direkt hydrolisiert. Die Phasen wurden getrennt und das Lösungsmittel der organischen Phase wurde am Rotationsverdampfer entfernt. Man erhielt 236 g 5-Phenyl-3-oxopentansäure-tert-butylester (95 %).

**Beispiel 3**

5-Methyl-3-oxohexansäureäthylester

26 g Acetessigsäureäthylester und 15 g Pyrrolidin wurden in 70 ml Toluol am Wasserabscheider gekocht, bis die theoretische Menge Wasser abgeschieden war. Diese Lösung wurde bei -40°C zu einer Suspension von 8,6 g Natriumamid in 300 ml flüssigem Ammoniak getropft. Durch Heizen auf Raumtemperatur wurde das Ammoniak entfernt. Nachdem die Reaktionslösung wieder auf 0°C gekühlt wurde, gab man bei der selben Temperatur 40,8 g Isopropyljodid gelöst in 100 ml Toluol zu und liess während 3 Stunden bei 0°C reagieren. Die Reaktionslösung wurde auf 200 ml Wasser gegossen, die Phasen wurden getrennt und das Toluol der organischen Phase wurde am Rotationsverdampfer entfernt. Vom Rückstand wurde dann bei 1,5 Torr [2 x $10^2$ Pa] und bis zu einer Kopftemperatur von 60°C 1,2 g Vorlauf abdestilliert, welcher vor allem 2-Isopropylacetessigsäureäthylester enthielt. Das zurückbleibende bräunliche Öl wurde in 70 ml Methylenchlorid gelöst, 21 g konzentrierte Salzsäure zugegeben und während 1,5 Stunden bei Raumtemperatur hydrolisiert. Die beiden Phasen wurden getrennt, die organische Phase wurde getrocknet, das Lösungsmittel entfernt und der Rückstand destilliert. Man erhielt 28,1 g 5-Methyl-3-oxohexansäureäthylester (82 %).

**Beispiel 4**

3-Oxopentansäureäthylester

Man verfuhr analog wie in Beispiel 1. Bei 40°C Reaktionsgemischtemperatur wurden dann 120 g Methylbromid durch ein Gaseinlassrohr in die Reaktionsmischung eingeleitet. Im weiteren wurden analog wie in Beispiel 1 vorgegangen. Man erhielt 127 g 3-Oxopentansäureäthylester (88 %).

| Beispiel | Edukt | $-NR_2$ | R''X | Lösungsmittel | Temp. °C | Produkt | Ausbeute |
|---|---|---|---|---|---|---|---|
| 5 | Acetessigsäure-methylester | Pyrrolidin | $CH_3Br$ | Diäthylenglykol-dimethyläther | -10 | 3-Oxopentansäure-methylester | 92 % |
| 6 | " | " | " | Toluol | -10 | " | 97 % |
| 7 | " | " | " | Tetrahydrofuran | -10 | " | 91 % |
| 8 | " | " | " | Diäthyläther | -10 | " | 87 % |
| 9 | " | " | " | Pyridin | -10 | " | 95 % |
| 10 | " | " | " | Hexan | -10 | " | 90 % |
| 11 | " | " | " | Benzol | 10 | " | 98 % |
| 12 | Acetessigsäure-äthylester | " | Benzylchlorid | Toluol | 30 | 5-Phenyl-3-oxopentan-säureäthylester | 99 % |
| 13 | " | " | Allylchlorid | Diäthyläther | -20 | 3-Oxohept-6-en-säure-äthylester | 94 % |
| 14 | " | " | Butylbromid | Diäthyläther | -20 | 3-Oxooctansäure-äthylester | 96 % |
| 15 | " | " | $CH_3J$ | Toluol | 0 | 3-Oxopentansäure-äthylester | 99 % |
| 16 | Acetessigsäure-piperidinamid | " | Benzylchlorid | Diäthyläther | -30 | 5-Phenyl-3-oxopentan-säurepiperidinamid | 99 % |

**Patentansprüche**

1. Verfahren zur Herstellung von 4-substituierten Acetessigsäurederivaten aus den entsprechenden Acetessigsäurederivaten, dadurch gekennzeichnet, dass man ein Acetessigsäurederivat der allgemeinen Formel

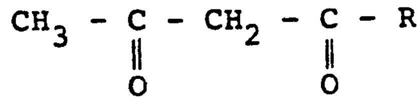

in welcher R = Alkoxyreste mit 1 bis 6 C-Atomen, ein Phenoxyrest, -$NR'_2$, wobei R' = Alkyl mit 1 bis 6 C-Atomen, Aryl oder $NR'_2$ = Azetidin, Pyrrolidin, Piperidin bedeutet, zunächst durch Behandeln mit einem sekundären Amin, bei erhöhter Temperatur und in Gegenwart eines organischen Lösungsmittel, unter Abscheidung des gebildeten Wassers, in das entsprechende 3-Enaminocarbonsäurederivat überführt, dieses durch Behandeln mit Natriumamid in flüssigem Ammoniak in das entsprechende Natriumsalz überführt, dieses durch Behandlung mit einer Halogen-Verbindung der Formel $R_1CH_2X$ oder $R_1R_2CHX$, worin $R_1$ und $R_2$ Alkyl-, Alkenyl-, Alkinyl-, Arylreste und X Chlor, Brom oder Jod bedeuten, in das entsprechende 4- substituierte Enaminoderivat überführt und aus diesem durch Hydrolyse das 4-substituierte Acetessigsäurederivat bildet.

2. Verfahren nach Patentanspruch 1, dadurch gekennzeichnet, dass man als sekundäres Amin Pyrrolidin oder Azetidin verwendet.

3. Verfahren nach Patentansprüchen 1 und 2, dadurch gekennzeichnet, dass man pro Mol Acetessigsäurederivat 1 bis 1,4 Mol Natriumamid verwendet.

4. Verfahren nach Patentansprüchen 1 bis 3, dadurch gekennzeichnet, dass man flüssiges Ammoniak auch als Lösungsmittel bei der Umsetzung mit der Halogenverbindung verwendet.

5. Verfahren nach Patentansprüchen 1 bis 3, dadurch gekennzeichnet, dass man nach der Bildung des Natriumsalzes das flüssige Ammoniak im Reaktionsgemisch belässt und bei der weiteren Reaktion mit der Halogenverbindung ein weiteres organisches Lösungsmittel verwendet.

6. Verfahren nach Patentansprüchen 1 bis 3, dadurch gekennzeichnet, dass man nach der Bildung des Natriumsalzes das flüssige Ammoniak abdestilliert und die weitere Reaktion mit der Halogenverbindung in Gegenwart eines organischen Lösungsmittels durchführt.

7. Verfahren nach Patentansprüchen 1 bis 3, dadurch gekennzeichet, dass auftretende 2-Substitutionsprodukte selektiv mit Wasser hydrolisiert und von den 4-Substitutionsprodukten abgetrennt werden.

**Claims**

1. Process for the preparation of 4-substituted acetoacetic acid derivatives from the corresponding acetoacetic acid derivatives, characterised in that one converts an acetoacetic acid derivative of the general formula

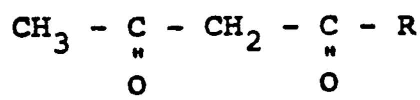

in which R = alkoxy radicals with 1 to 6 C-atoms, a phenoxy radical, -$NR'_2$, whereby R' = alkyl with 1 to 6 C-atoms, aryl or $NR'_2$ = azetidine, pyrrolidine, piperidine, first by treatment with a secondary amine at an elevated temperature and in the presence of an organic solvent, with separation of water formed, into the corresponding 3-enaminocarboxylic acid derivative, converts this by treatment with sodamide in liquid ammonia into the corresponding sodium salt, converts this by treatment with a halogen compound of the formula $R_1CH_2X$ or $R_1R_2CHX$, wherein $R_1$ and $R_2$ signify alkyl, alkenyl, alkynyl, aryl radicals and X chlorine, bromine or iodine, into the corresponding 4-substituted enamino derivative and forms the 4-substituted acetoacetic acid derivative from this by hydrolysis.

2. Process according to patent claim 1, characterised in that as secondary amine one uses pyrrolidine or azetidine.

3. Process according to patent claims 1 and 2, characterised in that, per mole of acetoacetic acid derivative, one uses 1 to 1.4 mole sodamide.

4. Process according to patent claims 1 to 3, characterised in that one uses liquid ammonia as solvent in the reaction with the halogen compound.

5. Process according to patent claims 1 to 3, characterised in that, after formation of the sodium salt, one leaves the liquid ammonia in the reaction mixture and, in the further reaction with the halogen compound, uses a further organic solvent.

6. Process according to patent claims 1 to 3, characterised in that, after formation of the sodium salt, one distills off the liquid ammonia and carries out the further reaction with the halogen compound in the presence of an organic solvent.

7. Process according to patent claims 1 to 3, characterised in that the 2-substitution products arising are selectively hydrolysed with water and separated from the 4-substitution products.

**Revendications**

1. Procédé pour la préparation de dérivés de l'acide acétoacétique substitué en position 4 à partir des dérivés de l'acide acétoacétique correspondants, caractérisé en ce qu'on transforme un dérivé de l'acide acétoacétique de formule générale

$$CH_3 - \underset{\underset{O}{\|}}{C} - CH_2 - \underset{\underset{O}{\|}}{C} - R$$

dans laguelle R = des radicaux alcoxy avec 1 à 6 atomes de C, un radical phénoxy, $-NR'_2$, où R' = alcoyle avec 1 à 6 atomes de C, aryle ou $NR'_2$ = azétidine, pyrrolidine, pipéridine, d'abord par traitement au moyen d'une amine secondaire, à température augmentée et en présence d'un solvant organique, avec séparation de l'eau formée, en le dérivé d'acide 3-énaminocarboxylique correspondant, en ce qu'on transforme celui-ci par traitement par l'amidure de sodium dans l'ammoniac liquide en le sel de sodium correspondant, en ce qu'on transforme celui-ci par traitement au moyen d'un composé halogéné de formule $R_1CH_2X$ ou $R_1R_2CHX$, où $R_1$ et $R_2$ représentent des radicaux alcoyle, alcényle, alcynyle, aryle et X représente chlore, brome ou iode, en le dérivé énamino substitué en position 4 correspondant et en ce qu'on forme le dérivé de l'acide acétoacétique substitué en position 4 à partir de celui-ci par hydrolyse.

2. Procédé selon la revendication 1, caractérisé en ce que l'on utilise comme amine secondaire la pyrrolidine ou l'azétidine.

3. Procédé selon les revendications 1 et 2, caractérisé en ce que l'on utilise 1 à 1,4 mole d'amidure de sodium par mole de dérivé de l'acide acétoacétique.

4. Procédé selon les revendications 1 à 3, caractérisé en ce qu'on utilise de l'ammoniac liquide également comme solvant lors de la réaction avec le composé halogéné.

5. Procédé selon les revendications 1 à 3, caractérisé en ce qu'après la formation du sel de sodium, on laisse l'ammoniac liquide dans le mélange réactionnel et lors de la réaction subséquente avec le composé halogéné on utilise un autre solvant organique.

6. Procédé selon les revendications 1 à 3, caractérisé en ce que, après la formation du sel de sodium, on chasse l'ammoniac liquide par distillation et on effectue la réaction subséquente avec le composé halogéné en présence d'un solvant organique.

7. Procédé selon les revendications 1 à 3, caractérisé en ce que les produits de substitution en position 2 formés sont hydrolysés sélectivement par l'eau et sont séparés des produits de substitution en position 4.